# EUROPÄISCHE PATENTSCHRIFT

(11) **EP 0 531 359 B1**
(45) Veröffentlichungstag und Bekanntmachung des Hinweises auf die Patenterteilung: **30.11.1994**
(21) Anmeldenummer: 91909833.5
(22) Anmeldetag: 21.05.1991
(51) Int. Cl.: C07C 309/17, C07C 303/44

(54) **VERFAHREN ZUR HERSTELLUNG HELLFARBIGER PASTEN VON ALPHA-SULFOFETTSÄUREALKYLESTER-ALKALIMETALLSALZEN**
PROCESS FOR PRODUCING LIGHT-COLOURED PASTES OF ALPHA-SULPHO FATTY ACID ALKYL ESTER ALKALI METAL SALTS
PROCEDE DE PRODUCTION DE PATES DE COULEUR CLAIRE EN SELS DE METAUX ALCALINS-ALKYLESTERS D'ACIDES GRAS ALPHA-SULFONIQUES

(30) Priorität: 30.05.1990 DE 4017466
(43) Veröffentlichungstag der Anmeldung: 17.03.1993
(73) Patentinhaber: Henkel Kommanditgesellschaft auf Aktien, 40191 Düsseldorf (DE)
(72) Erfinder: COLIGNON, Dietmar, D-4006 Erkrath (DE); DORRA, Erich, D-4000 Düsseldorf 13 (DE); LEPPER, Herbert, D-4010 Hilden (DE); PANTHEL, Günter, D-5657 Haan (DE); PIERRON, François, F-74000 Annecy (FR); SCHMIDT, Wolfgang, D-4019 Monheim 2 (DE); WREDE, Norbert, D-4000 Düsseldorf 13 (DE)
(86) Internationale Anmeldenummer: EP9100941
(87) Internationale Veröffentlichungsnummer: WO9118872

(56) Entgegenhaltungen:
- DE-A- 2 400 538
- DE-B- 1 185 178

## Beschreibung

Die Erfindung betrifft ein Verfahren zur Herstellung von hellfarbigen Pasten von α-Sulfofettsäurealkylester-Alkalimetallsalzen, bei dem man zur Bleichung des Sulfonierungsproduktes Wasserstoffperoxid als alleiniges Bleichmittel während der Neutralisation des α-Sulfofettsäurealkylesters zusetzt.

α-Sulfofettsäurealkylester-Alkalimetallsalze haben derzeit steigende Bedeutung als oberflächenaktive Substanzen für Wasch- und Reinigungsmittel aus nachwachsenden natürlichen Rohstoffen. Man erhält die α-Sulfofettsäurealkylester-Alkalimetallsalze nach bekannten Verfahren in Form von wässrigen Lösungen oder Pasten durch Neutralisation von α-Sulfofettsäurealkylestern, welch letztere durch Umsetzung von Fettsäureniedrigalkylestern mit gasförmigem SO₃ synthetisiert werden können. Als Basis für die Herstellung der α-Sulfofettsäurealkylester-Alkalimetallsalze, dienen letzten Endes Fette und Öle natürlichen Ursprungs, aus denen die Fettsäureniedrigalkylester durch Fettspaltung und nachfolgende Veresterung der freien Fettsäuren mit niederen Alkanolen oder durch Umesterung der natürlichen Triglyceride mit niederen Alkanolen erhalten werden. Bei beiden Reaktionen ist Methanol als niederes Alkanol bevorzugt. Die Fettsäureniedrigalkylester stellen Gemische dar, in denen Fettsäurereste mit 6 bis 22 Kohlenstoffatomen vorkommen, wobei die Kettenlängenverteilung vom Ursprung der natürlichen Fette oder Öle abhängig ist.

Diese Fettsäureestergemische werden häufig nicht als solche, sondern in Form bestimmter Fraktionen zur Synthese eingesetzt. Durch Sulfonierung der Fettsäureestergemische mit gasförmigem SO₃ erhält man saure α-Sulfofettsäurealkylester, die durch Neutralisation auf einen pH-Wert von 6 bis 8 in wässrige Pasten von α-Sulfofettsäurealkyleseter-Alkalimetallsalzen überführt werden.

Die rohen α-Sulfofettsäurealkylester und gegebenenfalls auch ihre Alkalimetallsalze stellen mehr oder weniger gefärbte Produkte dar, die in der Regel vor und/oder nach der Neutralisation einer Behandlung mit üblichen Bleichmitteln unterworfen werden müssen. Im Zusammenhang mit der Herstellung von hellfarbigen α-Sulfofettsäurealkylester-Alkalimetallsalzlösungen und -pasten sind unterschiedliche Bleichverfahren bekannt geworden. Für eine effektive Produktaufhellung sind Kombinationsbleichverfahren entwickkelt worden. Nach der DE-AS 12 34 709 wird in einer ersten Bleichstufe zunächst der saure α-Sulfofettsäurealkylester mit wässriger Wasserstoffperoxidlösung behandelt. Anschließend wird das teilgebleichte Sulfonierungsprodukt neutralisiert, bevor es in einer zweiten Bleichstufe der Einwirkung von weiterer Wasserstoffperoxidlösung oder von wässriger Hypochloritlösung ausgesetzt wird. Nach der DE-OS 33 19 591 wird das neutralisierte Sulfonierungsprodukt zunächst bei pH-Werten zwischen 7 und 10 mit wässriger Hypochloritlösung gebleicht. Im Anschluß daran wird bei pH-Werten von ≦ 7 wässrige Wasserstoffperoxidlösung zugesetzt, um die erreichten Farbwerte zu stabilisieren. Diese Verfahren sind mit dem Nachteil behaftet, daß die bei der Neutralisation erreichten und erwünschten hohen Waschaktivsubstanzgehalte der α-Sulfofettsäurealkylester-Alkalimetallsalzpasten durch das Einbringen der wässrigen Bleichmittellösungen wieder herabgesetzt werden. Unter Waschaktivsubstanz (WAS) wird im Zusammenhang mit der Erfindung die Summe aus α-Sulfofettsäurealkylester-Alkalimetallsalz und dem stets als Nebenprodukt vorhandenen α-Sulfofettsäureester verstanden.

Eine besondere Schwierigkeit bei der Herstellung und Handhabung von wässrigen α-Sulfofettsäurealkylester-Alkalimetallsalzpasten ergibt sich aus ihrem Viskositätsverhalten in Abhängigkeit von der Waschaktivsubstanzkonzentration. Die nach den gängigen technischen Verfahren hergestellten α-Sulfofettsäurealkylester-Alkalimetallsalze, im Folgenden auch kurz als Estersulfonate bezeichnet, bilden in wässrigen Zusammensetzungen nur bei WAS-Gehalten bis zu etwa 40 Gew.-% und dann wieder ab Feststoffgehalten von etwa 55 Gew.-% Lösungen oder Suspensionen mit so geringer Viskosität, daß eine hinreichende Beweglichkeit gegeben ist, um einen störungsfreien Ablauf technischer Vorgänge zu gewährleisten. In dem dazwischen liegenden Konzentrationsbereich, also bei WAS-Gehalten von etwa 40 bis 55 Gew.-% zeigen die wässrigen Estersulfonat-Zusammensetzungen extrem hohe Viskositätswerte und stellen sich als mehr oder weniger feste Gele dar, die nicht gerührt oder umgepumpt werden können. Die Unter- und Obergrenzen der individuellen Viskositätsmaxima können im übrigen jeweils um ± 5 Gew.-%. WAS-Gehalt schwanken. Dieses besondere Konzentrations-/Viskositätsverhalten bedingt, daß durch einfache Neutralisation der sauren α-Sulfofettsäurealkylester mit der berechneten Menge wässriger Alkalimetallhydroxidlösungen nach den bekannten Verfahren keine Estersulfonatpasten mit WAS-Gehalten über 35 bis 40 Gew.-% eingestellt werden können. Nach dem Überschreiten der Untergrenze des Viskositätsmaximums geht die Rührbarkeit und Vermischbarkeit des reagierenden Gemisches verloren. Die mangelnde Rühr- und Mischbarkeit verhindert einen hinreichenden schnellen Abtransport der Neutralisationswärme. Durch lokale Konzentrations- und Temperaturspitzen werden unerwünschte Nebenreaktionen ausgelöst, insbesondere die Spaltung der in den Estersulfonaten vorhandenen Esterbindungen, wodurch im Endprodukt unerwünscht hohe Konzentrationen an Alkalimetall-Disalzen der freien α-Sulfofettsäuren entstehen. Selbstverständlich ist auch die weitere Verarbeitung von Estersulfonatpasten, die durch den hohen Viskositätsanstieg immobilisiert sind, bis hin zur Undurchführbarkeit beeinträchtigt, allein schon durch die Tatsache, daß derartige wässrige Zusammmensetzungen nicht mehr gieß- und pumpfähig sind.

Das Entstehen von Disalzen der freien α-Sulfofettsäurealkylester ist aus mehreren Gründen unerwünscht. Die Disalze sind in Wasser nur im beschränkten Umfang löslich und zeigen darüber hinaus unzureichende oberflächenaktive Eigenschaften. Vor allem aber haben Disalze als Nebenprodukte in Estersulfonatpasten eine beträchtliche viskositätssteigernde Wirkung.

Es hat in der Vergangenheit nicht an Versuchen gefehlt, die durch das besondere Konzentrations-/Viskositätsverhalten der Estersulfonate und das unerwünschte Entstehen von α-Sulfofettsäure-Disalzen bedingten nachteiligen Erscheinugen wenigstens weitgehend ausschalten. So wurde vorgeschlagen, das Fließverhalten von wässrigen Estersulfonat-Zusammensetzungen durch den Zusatz von Fließhilfsmitteln zu verbessern. Nach der DE-OS 33 05 430 werden als Viskositätsregler aliphatische Alkohole mit 8 bis 40 Kohlenstoffatomen und 1 bis 6 Hydroxylgruppen, Alkylphenole und Anlagerungsprodukte von bis zu 20 Mol Ethylenoxid und/oder Propylenoxid an die genannten Alkohole und Alkylphenole eingesetzt.

Im Zusammenhang mit der unerwünschten Bildung von Disalzen bei der Aufarbeitung der saueren α-Sulfofettsäurealkylester beschreibt die DE-OS 31 23 681 ein Verfahren, bei dem die Neutralisation in zwei Stufen durchgeführt wird. In der ersten Neutralisationsstufe wird dort mit einer 15 bis 50 gew.-%igen Alkalimetallhydroxidlösung in Gegenwart eines Alkohols mit 1 bis 4 Kohlenstoffatomen, vorzugsweise Methanol, in einer Menge von 5 bis 20 Gew.-%, bezogen auf das Gewicht des sulfonierten Produktes, bis auf einen pH-Wert von 2,5 bis 4 neutralisiert, bevor in der zweiten Neutralisationsstufe mit Hilfe einer stärker verdünnten Alkalimetallhydroxidlösung ein End-pH-Wert von 6 bis 7 eingestellt wird. Mit Hilfe dieses Verfahrens soll es möglich sein, in den Estersulfonat-Zusammensetzungen den Disalzgehalt auf 5 Gew.-%, bezogen auf Waschaktivsubstanz, oder weniger zurückzudrängen. Ein gravierender Nachteil dieses Verfahrens liegt auf der Hand: Die auf diese Weise hergestellten Estersulfonatpasten enthalten beträchtliche Mengen Alkohol, die bei der Herstellung von Waschmittelgemischen durch Sprühtrocknung insofern stören, als sie das unerwünschte "Pluming" auslösen können. Zur Begrenzung des Alkoholgehaltes in den Endprodukten schlägt die DE-OS 33 34 517 vor, die gegebenenfalls durchgeführte Bleiche und die Neutralisation der rohen α-Sulfofettsäurealkylester in Gegenwart einer solchen Menge eines niederen Alkohols vorzunehmen, daß eine wässrige Aufschlämmung mit 30 bis 40 Gew.-% und - bezogen auf das Gewicht des α-Sulfofettsäureestersalzes - 5 bis 15 Gew.-% eines niederen Alkoholsulfates sowie 8 bis 40 Gew.-% des niederen Alkohols erhalten wird. Abschließend soll die wässrige Aufschlämmung derart eingeengt werden, daß sie 40 bis 65 Gew.-% α-Sulfofettsäureestersalz, 2 bis 10 Gew.-% niederes Alkoholsulfat und höchstens 2 Gew.-% niederen Alkohol enthält.

Nach der DE-OS 34 32 324 läßt sich der Disalzgehalt in α-Sulfofettsäurealkylester-Alkalimetallsalz-Pasten dadurch regeln und senken, daß man das rohe Sulfonierungsprodukt vor der Behandlung mit einem wässrigen Medium einer Umesterungsreaktion unterwirft, bei der - bezogen auf den zur α-Sulfonierung nicht verbrauchten SO₃-Anteil - wenigstens 0,5 Mol-Äquivalente Alkohol eingesetzt werden. Nach der DE-OS 35 38 910 können α-Sulfofettsäurealkylester-Salzpasten mit Feststoffgehalten oberhalb von 35 Gew.-% dadurch hergestellt werden, daß man die rohen Estersulfonate einer Umesterrnng im Sinne der DE-OS 34 32 324 unterwirft und dann bei der anschließenden Aufarbeitung durch Neutralisation mit oder ohne vorhergehende oder nachfolgende Bleiche in den wässrigen Pasten Feststoffgehalte von mehr als 35 Gew.-% einstellt.

Die Erfindung geht auf die Aufgabenstellung zurück, ein Verfahren aufzufinden, daß es gestattet, die bei der Herstellung von α-Sulfofettsäurealkylester-Alkalimetallsalzpasten erforderliche Bleichung der Sulfonierungsprodukte mit wässrigen Bleichmittellösungen so zu gestalten, daß der Feststoffgehalt der Pasten durch die Bleichung nicht herabgesetzt wird. Es sollte insbesondere ein Verfahren entwickelt werden, bei dem ohne Zusatz von Fremdstoffen wie längerkettigen aliphatischen Mono- und Polyalkoholen und deren Alkylenoxidanlagerungsprodukten oder von kurzkettigen Alkoholen durch direkte Neutralisation der sauren α-Sulfofettsäurealkylester mit wässrigen Alkalimetallhydroxidlösungen und eine darauf abgestimmte Bleichung fließ- und pumpfähige Estersulfonatpasten mit WAS-Gehalten von 60 bis 70 Gew.-% hergestellt werden können.

Die Lehre der Erfindung geht von der überraschenden Erkenntnis aus, daß hellfarbige Pasten von α-Sulfofettsäurealkylester-Alkalimetallsalzen ohne Verminderung des WAS-Gehaltes im Endprodukt erhalten werden können, wenn an Wasserstoffperoxid als alleiniges Bleichmittel bei der Neutralisation des sauren Sulfonierungsproduktes zusetzt und das Neutralisationsprodukt bei erhöhter Temperatur nachreagieren läßt. Weiterhin wurde gefunden, daß hellfarbige α-Sulfofettsäurealkylester-Alkalimetallsalzpasten mit WAS-Gehalten von 60 bis 70 Gew.-% ohne viskositätsregulierende Zusätze dadurch erhalten werden können, daß man bei der Neutralisation das saure Sulfonierungsprodukt gleichzeitig mit wässriger Alkalimetallhydroxidlösung und wässriger Wasserstoffperoxidlösung in eine bereits vorliegende wässrige Phase einträgt und dabei dafür sorgt, daß der pH-Wert der wässrigen Phase sich nur in einem ganz bestimmten Bereich bewegt. Dies wird erreicht, indem man den pH-Wert der wässrigen Phase durch Variation der Volumenströme von α-Sulfofettsäurealkylester und wässriger Alkalimetallhydroxidlösung steuert.

Gegenstand der Erfindung ist ein Verfahren zur Herstellung hellfarbiger Pasten von α-Sulfofettsäurealkylester-Alkalimetallsalzen durch Umsetzen von Fettsäurealkylestern mit gasförmigem SO₃, anschließende Nachreaktion in flüssiger Phase, Neutralisation mit wässrigen Alkalimetallhydroxidlösungen und Behandeln mit Wasserstoffperoxid, bei dem man das Wasserstoffperoxid während der Neutralisation der α-Sulfofettsäurealkylester zusetzt und das enthaltene Neutralisationsprodukt einer temperaturkontrollierten Nachreaktion unterwirft.

Erfindungsgemäß setzt man während der Neutralisation 0,5 bis 3 Gew.-%, vorzugsweise 1,5 bis 2,5 Gew.-% Wasserstoffperoxid, bezogen auf Waschaktivsubstanz im Neutralisationsprodukt, zu. Wasserstoffperoxid wird dabei als 100 gew.-%ige Substanz berechnet. Das Wasserstoffperoxid wird in Form von 5 bis 35 gew.-%igen, vorzugsweise 10 bis 20 gew.-%igen wässrigen Lösungen zugesetzt. Die temperaturkontrollierte Nachreaktion des Neutralisationsproduktes wird vorzugsweise bei 60 bis 90 °C durchgeführt.

In einer besonderen Ausführungsform der Erfindung wird während der Neutralisation oder nach der Neutralisation, jedoch vor der temperaturkontrollierten Nachreaktion mindestens ein üblicher Bleichaktivator zugesetzt. Als Bleichaktivatoren eignen sich insbesondere organische Verbindungen, die unter den Bedingungen der Neutralisation und der temperaturkontrollierten Nachreaktion mit Wasserstoffperoxid Percarbonsäuren oder Percarbonsäureanionen bilden können. Hier sind vor allem Verbindungen geeignet, die N-Acylgruppen, vorzugsweise N-Acetylgruppen enthalten. Spezielle Beispiele für bevorzugte Bleichaktivatoren sind Tetraacetylethylendiamin und Tetraacetylglykoluril. Die Bleichaktivatoren werden dem Reaktionsgemisch in Mengen von 0,1 bis 5 Gew.-%, bezogen auf die im Neutralisationsprodukt zu erwartende Waschaktivsubstanz, zugesetzt.

Bei der temperaturkontrollierten Nachreaktion hält man das Wasserstoffperoxid und gegebenenfalls Bleichaktivator enthaltende Neutralisationsprodukt vorzugsweise so lange auf der vorgesehenen Temperatur im Bereich von 60 bis 90°C, bis es eine Klett-Farbzahl ≦ 200, vorzugsweise ≦ 100 (gemessen an einer 5 Gew.-% Waschaktivsubstanz enthaltenden wässrigen Lösung in einer 4 cm-Küvette mit Blaufilter 420 nm) aufweist.

In bestimmten Fällen, insbesondere dann, wenn stärker gefärbte Sulfonierungsprodukte zu verarbeiten sind, kann es angebracht sein, dem Neutralisationsprodukt vor Beginn der Nachreaktion noch einmal Wasserstoffperoxid zuzuführen. In diesem Fall werden in der Regel weitere 0,1 bis 2 Gew.-%, vorzugsweise 0,1 bis 0,5 Gew-%, bezogen auf vorhandene Waschaktivsubstanz, zugesetzt.

Im Rahmen des erfindungsgemäßen Verfahrens ist es weiterhin im Zusammenhang mit der Herstellung von hochkonzentrierten α-Sulfofettsäurealkylester-Alkalimetallsalzpasten, insbesondere von solchen mit WAS-Gehalten von 60 bis 70 Gew.-% bevorzugt, bei der Neutralisation des sauren α-Sulfofettsäureesters das Wasserstoffperoxid und den gegebenenfalls eingesetzten Bleichaktivator gleichzeitig mit dem Sulfonierungsprodukt und der wässrigen Alkalimetallhydroxidlösung unter Aufrechterhaltung eines pH-Wertes im Bereich von 2 bis 8 in eine 0 bis 70 Gew.-% Waschaktivsubstanz enthaltende wässrige Phase einzuleiten und WAS-Gehalte von 60 bis 70 Gew.-%, vorzugsweise 60 bis 65 Gew.-%, einzustellen.

In einer speziellen Ausführungsform der Erfindung werden Wasserstoffperoxid, Sulfonierungsprodukt, wässrige Alkalimetallhydroxidlösung und gegebenenfalls Bleichaktivator gleichzeitig in eine wässrige Phase, die am Anfang 0 Gew.-% Waschaktivsubstanz enthält, also aus Wasser besteht, eingeleitet.

Werden bei Beginn der Neutralisation als wässrige Phase Lösungen eingesetzt, die einen Gehalt an Waschaktivsubstanz aufweisen, der von 0 verschieden ist und bis zu 55 Gew.-% betragen kann, so werden diese Lösungen vorher auf einen pH-Wert im Bereich von 2 bis 8 eingestellt.

In einer bevorzugten Ausführungsform der Erfindung wird während des Neutralisationsvorgangs in der wässrigen Phase bis zum Erreichen eines Gehaltes an Waschaktivsubstanz von 55 bis 65 Gew.-%, vorzugsweise von 60 bis 65 Gew.-%, ein pH-Wert im Bereich von 2 bis 6, vorzugsweise im Bereich von 3 bis 5, aufrechterhalten. Weiterhin ist es bevorzugt, nach dem Erreichen eines Gehaltes an Waschaktivsubstanz von 55 bis 65 Gew.-%, vorzugsweise von 60 bis 65 Gew.-%, in der wässrigen Phase einen pH-Wert von 5 bis 8, vorzugsweise im Bereich von 5,5 bis 7,5 einzustellen und aufrechtzuerhalten.

In einer weiteren bevorzugten Ausführungsform der Erfindung werden Wasserstoffperoxid, Sulfonierungsprodukt, wässrige Alkalimetallhydroxidlösung und gegebenenfalls Bleichaktivator gleichzeitig in eine wässrige Phase eingeleitet, die am Anfang mindestens 55 Gew.-% Waschaktivsubstanz enthält. In diesem Fall wird während des Neutralisationsvorgangs in der wässrigen Phase vorzugsweise ein pH-Wert im Bereich von 5,5 bis 7,5 aufrechterhalten.

Die Neutralisation der α-Sulfofettsäurealkylester wird bei Temperaturen unterhalb von 95 °C, vorzugsweise bei Temperaturen im Bereich von 60 bis 80 °C durchgeführt.

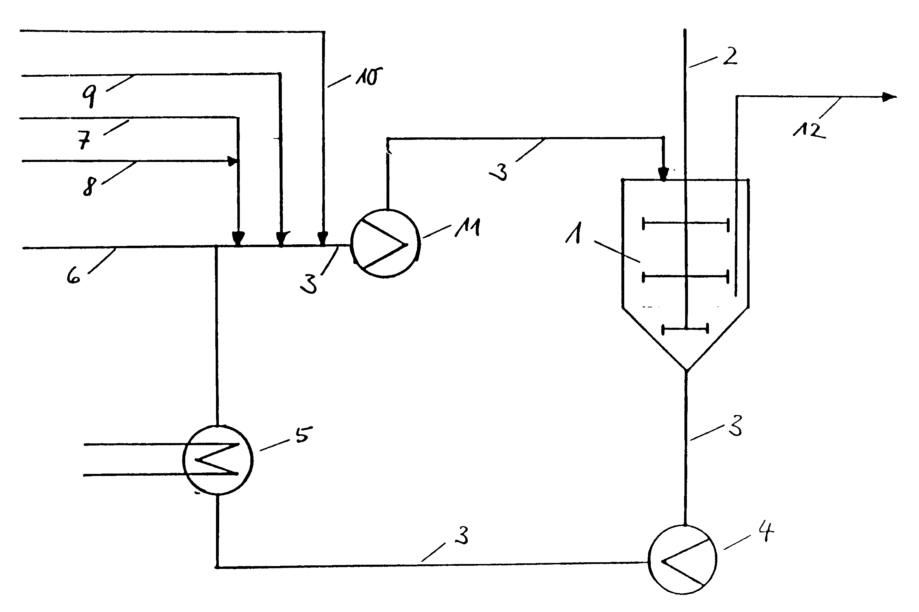

Die Neutralisation der sauren α-Sulfofettsäurealkylester wird zweckmäßigerweise in einer Neutralisationsschleife durchgeführt, deren schematischer Aufbau in der Fig. 1 dargestellt ist. Der überwiegende Teil der wässigen Phase befindet sich in dem Rührbehälter 1 und wird mit Hilfe des Rührers 2 ständig durchmischt. Über die Kreislaufleitung 3 wird daraus mittels der Umwälzpumpe 4 wässrige Phase laufend abgezogen und in dem für die Steuerung der Reaktionstemperatur vorgesehenen Kühler 5 im erforderlichen Ausmaß gekühlt. Der zu neutralisierende α-Sulfofettsäurealkylester wird über die Leitung 6 in den Strom der umgepumpten wässrigen Phase eingeleitet. Wässrige Alkalimetallhydroxidlösung einer Standard-Konzentration, beispielsweise 50 gew.-%ige Natriumhydroxidlösung, wird dem Kreislauf über die Leitung 7 zugeführt. Durch Zufuhr von Wasser über die Leitung 8 kann die Konzentration der Standard-Alkalilitallhydroxidlösung vor deren Einspeisung in den Produktkreislauf auf den akut geforderten Wert vermindert werden. Wässrige Wasserstoffperoxidlösung kann über die Leitung 9 in den Neutralisationskreislauf eingespeist werden. Gegebenenfalls zugesetzte Bleichaktivatoren können über die Leitung 10 in die zirkulierende wässrige Phase eingebracht werden. Das Gemisch aus saurem α-Sulfofettsäurealkylester, Alkalimetallhydroxidlösung und im Kreislauf geführter wässriger Phase gelangt dann zur weiteren Homogenisierung in den Mischer 11 und wird von dort über den letzten Abschnitt der Kreislaufleitung 3 in den Rührbehälter 1 gefördert. Die bei der Neutralisation entstehende α-Sulfofettsäurealkylester-Alkalimetallsalzpaste kann über die Leitung 12 abgezogen werden. Eine Neutralisationsschleife des hier beschriebenen Typs kann ausschließlich aus üblichen Vorrichtungen, Armaturen und Leitungen aufgebaut werden. Die erforderliche Überwachung von pH und Reaktionstemperatur und die Regulierung der Produkt- und Kühlmittelströme kann nach bekannten Verfahren der Meß- und Regel technik für chemische Verfahren erfolgen.

Die auf die Neutralisation nachfolgende Nachreaktion kann in einem heizbaren, mit Rührwerk versehenen Edelstahlbehälter bekannter Bauart durchgeführt werden. Das Neutralisationsprodukt wird zweckmäßigerweise nach dem Verlassen der Neutralisationsschleife in einem unter vermindertem Druck, beispielsweise 300 bis 400 mbar, stehenden Behälter entschäumt, bevor es in die Nachreaktionsvorrichtung eingebracht wird.

Unter Verwendung der beschriebenen Neutralisationsschleife kann die Neutralisation in einfacher Weise für den kontinuierlichen Betrieb eingerichtet werden. In diesem Falle besteht die im Neutralisationskreislauf umgepumpte wässrige Phase aus einer α-Sulfofettsäurealkylester-Alkalimetallsalzpaste, die in ihrem WAS-, Wasserstoffperoxid- und gegebenenfalls Bleichaktivatorgehalt mit dem aus dem Kreislauf abgezogenen Neutralisationsprodukt übereinstimmt. Saurer α-Sulfofettsäurealkylester, Alkalimetallhydroxidlösung, Wasserstoffperoxidlösung und gegebenenfalls Bleichaktivator werden dem Neutralisationskreislauf in dem Maße zugeführt, wie Neutralisationsprodukt aus dem Kreislauf abgezogen wird. Zur Aufrechterhaltung eines konstanten WAS-Gehaltes wird die zugeführte Standard-Alkalimetallhydroxidlösung durch Zumischen von Wasser auf die dafür erforderliche Konzentration verdünnt.

Unter Fettsäurealkylestern im Sinne der Erfindung werden Niedrigalkylester von gesättigten Fettsäuren verstanden, insbesondere Ester von Fettsäuren mit 10 bis 18 Kohlenstoffatomen und gesättigten aliphatischen Alkoholen mit 1 bis 4 Kohlenstoffatomen. Grundsätzlich kann man von einzelnen Fettsäurealkylestern ausgehen. In der Regel verwendet man als Ausgangsmaterial jedoch Estergemische, wie sie aus Fetten und Ölen natürlichen Ursprungs entweder durch Esterspaltung und nachfolgende Veresterung mit niederen Alkanolen oder durch Umesterung mit niederen Alkanolen nach bekannten Verfahren erhältlich sind, wobei die entsprechenden Fettsäuremetylestergemische wiederum bevorzugt sind. Soweit die auf diese Weise erhaltenen Fettsäureestergemische größere Anteile an Estern von Fettsäuren mit weniger als 10 Kohlenstoffatomen enthalten, werden diese "Vorlauffettsäureester" in der Regel durch Destillation abgetrennt. Die Fettsäureester dürfen außer der CH₂-Gruppe in α-Stellung zur Estergruppierung keine sulfatierbaren oder sulfonierbaren Gruppen enthalten. Aus diesem Grund kommen Hydroxyfettsäureester oder Hydroxyfettsäureester enthaltende Gemische nicht als Ausgangsmaterial in Betracht. Fettsäureestergemische, die nicht zu vernachlässigende Mengen an Estern ungesättigter Fettsäuren enthalten, insbesondere solche Ester, die eine Jodzahl über 5 aufweisen, sind erst nach einer Absättigung der Doppelbindungen im Zuge einer hydrierenden Härtung nach bekannten Verfahren als Ausgangsmaterial geeignet. Bei der hydrierenden Härtung werden die Jodzahlwerte der Estergemische vorzugsweise auf Werte von 0,2 und kleiner vermindert.

Die Sulfonierung der Fettsäureester erfolgt mit gasförmigem SO₃ als Sulfonierungsreagenz bei Temperaturen von 30 bis 100 °C. Dabei wird das SO₃ mit Luft oder Stickstoff verdünnt, vorzugsweise in Form eines Gasgemisches mit 1 bis 10 Vol.-% SO₃ mit den Fettsäureestern in Berührung gebracht. Die Menge des SO₃ wird so bemessen, daß das Molverhältnis von Fettsäureester : SO₃ im Bereich von 1 : 1,2 bis 1 : 1,8 liegt. Diese Umsetzung kann in üblichen, für die Sulfonierung von organischen Verbindungen wie Fettalkoholen, Alkylbenzolen oder Olefinen geeigneten Reaktoren, insbesondere in Fallfilmreaktoren oder mehrstufigen Rührkesselkaskaden durchgeführt werden.

Das aus dem Sulfierreaktor kommende rohe Sulfonierungsprodukt weist noch nicht den gewünschten Sulfonierungsgrad auf. Aus diesem Grund wird das rohe Reaktionsprodukt unmittelbar nach der Sulfonierung einer geeigneten Vorrichtung zugeführt, in der es unter mechanischer Bewegung über einen Zeitraum von 20 bis 40 Minuten, vorzugsweise 25 bis 35 Minuten, einer temperaturkontrollierten Nachreaktion überlassen wird, bis der gewünschte Sulfiergrad erreicht ist. Die für diesen Reaktionsschritt notwendige Vorrichtung kann aus einem üblichen Reaktor mit Heiz- und Kühlkreislauf, einer üblichen temperierbaren Rohrschlange oder einer üblichen Rührkesselkaskade bestehen. Die Nachreaktion wird bei Temperaturen von 60 bis 100 °C durchgeführt. Die mechanische Bewegung des sulfonierten Produktes während der Nachreaktion kann durch Rühren, durch Zufuhr des Produktes unter Druck, durch den Einbau von Umlenkschikanen in die Vorrichtung oder, bei Verwendung einer Rohrschlange, durch Ausbildung einer turbulenten Strömung bewirkt werden. Die Nachreaktion des sulfonierten Produktes kann durch geeignete Wahl der genannten Parameter, insbesondere der Reaktionszeit, so gesteuert werden, daß ein Sulfiergrad von mindestens 90 %, vorzugsweise 94 bis 98 % erreicht wird.

Im Anschluß an diese Nachreaktion wird das gealterte Sulfonierungsprodukt der Neutralisation und Bleichung gemäß der Erfindung zugeführt.

### Beispiele

### Beispiel 1

Als Ausgangsmaterial wurde ein technischer Palmitin-/Stearinsäuremethylester (in Gew.-% nach der Kettenlänge im Fettsäurerest: 0,2 C₁₂; 1,2 C₁₄; 61,4 C₁₆; 0,9 C₁₇; 35,9 C₁₈; 0,4 C₂₀; mittleres Molgewicht 281,5; Säurezahl 1,1; Jodzahl 0,1; Verseifungszahl 202,1) verwendet. Der Fettsäuremethylester wurde kontinuierlich in einem üblichen Fallfilmreaktor bei 80 °C mit einem SO₃-Luftgemisch (5 Vol.-% SO₃) im Molverhältnis 1 : 1,25 sulfoniert. Das resultierende Reaktionsgemisch wurde in einer aus vier Rührkesseln bestehenden Verweilzeitkaskade mit einer Verweilzeit von 25 Minuten einer Nachreaktion unterworfen. Danach betrug die Säurezahl des Sulfonierungsproduktes 198. Der Sulfonierungsgrad lag bei 96 %.

In einer Neutralisationsschleife des beschriebenen Typs wurden 616 kg Wasser eingebracht und umgepumpt. In den Kreislauf der wässrigen Phase wurden 6836 kg des vorstehend beschriebenen gealterten Sulfonierungsproduktes und 1890 kg 50 gew.-%igen Natriumhydroxidlösung zunächst mit einer solchen Geschwindigkeit eingespeist, daß in der wässrigen Phase ein pH-Wert von 5 aufrechterhalten wurde. Sobald der WAS-Gehalt der wässrigen Phase 55 Gew.-% erreicht hatte, wurden die Zuflußgeschwindigkeiten des Sulfonierungsproduktes und der Natriumhydroxidlösung so einreguliert, daß der pH-Wert in der wässrigen Phase bei 6 lag. Parallel zur Einspeisung des Sulfonierungsproduktes und der wässrigen Natriumhydroxidlösung wurden 658 kg 20 gew.-%ige wässrige Wasserstoffperoxidlösung (2 Gew.-% bezogen auf zu erwartende Waschaktivsubstanz) in den Neutralisationskreislauf eingebracht. Nachdem das gesamte saure Sulfonierungsprodukt dem Neutralisationskreislauf zugeführt war, wurde der pH-Wert der wässrigen Phase durch Zugabe der restlichen Menge Natriumhydroxidlösung auf 6,1 angehoben. Während der gesamten Neutralisation wurde die Reaktionstemperatur auf 90 bis 93 °C gehalten. Die wässrige Phase konnte während des Neutralisationsvorgangs jederzeit problemlos gerührt und umgepumpt werden.

Das neutralisierte Produkt wurde in einen geschlossenen Behälter abgepumpt und bei vermindertem Druck (etwa 380 mbar) entschäumt, bevor es in einen mit Rührwerk versehenen Edelstahlbehälter überführt und 38 Stunden lang unter Durchmischung einer Temperatur von 75 °C ausgesetzt wurde. Der zeitliche Verlauf der Aufhellung wurde anhand von Messungen der Klett-Farbzahlen von entnommenen Proben verfolgt. Die Klett-Farbzahlen wurden an 5 Gew.-% Waschaktivsubstanz enthaltenden wässrigen Lösungen in einer 4 cm-Küvette unter Verwendung eines Blaufilters 420 nm gemessen. Der zeitliche Verlauf der Bleichung ist in der Tabelle 1 wiedergegeben.

**Tabelle 1**

| Aufhellung während der Bleichreaktion bei 75 °C | |
|---|---|
| Reaktionszeit (n) | Klett-Farbzahl |
| 0 | 640 |
| 12 | 232 |
| 16 | 150 |
| 20 | 130 |
| 38 | 80 |

Es wurden 10 000 kg rühr- und pumpfähige α-SulfofettsäuremethylesterNatriumsalzpaste mit einem Waschaktivsubstanzgehalt von 66,0 Gew.-% (54 Gew.-% α-Sulfofettsäuremethylester-Natriumsalz und 12 Gew.-% α-Sulfofettsäuredinatriumsalz) erhalten.

## Patentansprüche

1. Verfahren zur Herstellung hellfarbiger Pasten von α-Sulfofettsäurealkylester-Alkalimetallsalzen durch Umsetzen von Fettsäurealkylestern mit gasförmigem SO₃, anschließende Nachreaktion in flüssiger Phase, Neutralisation mit wässrigen Alkalimetallhydroxidlösungen und Behandeln mit Wasserstoffperoxid, dadurch gekennzeichnet, daß man das Wasserstoffperoxid während der Neutralisation der α-Sulfofettsäurealkylester zusetzt und das enthaltene Neutralisationsprodukt einer temperaturkontrollierten Nachreaktion unterwirft.

2. Verfahren nach Anspruch 1, dadurch gekennzeichnet, daß man bei der Neutralisation 0,5 bis 3 Gew.-%, vorzugsweise 1,5 bis 2,5 Gew.-% Wasserstoffperoxid, bezogen auf Waschaktivsubstanz im Neutralisationsprodukt, zusetzt.

3. Verfahren nach mindestens einem der Ansprüche 1 und 2, dadurch gekennzeichnet, daß man das Wasserstoffperoxid als 5 bis 35 gew.-%ige Lösung, vorzugsweise als 10 bis 20 gew.-%ige Lösung zusetzt.

4. Verfahren nach mindestens einem der Ansprüche 1 bis 3, dadurch gekennzeichnet, daß man das Neutralisationsprodukt einer Nachreaktion bei 60 bis 90 °C unterwirft.

5. Verfahren nach mindestens einem der Ansprüche 1 bis 4, dadurch gekennzeichnet, daß man während der Neutralisation mindestens einen Bleichaktivator zusetzt.

6. Verfahren nach mindestens einem der Ansprüche 1 bis 4, dadurch gekennzeichnet, daß man nach beendeter Neutralisation und vor der temperaturkontrollierten Nachreaktion mindestens einen Bleichaktivator zusetzt.

7. Verfahren nach Anspruch 5 oder 6, dadurch gekennzeichnet, daß man als Bleichaktivator mindestens eine organische Verbindung zusetzt, die unter den Bedingungen der Neutralisation und der temperaturkontrollierten Nachreaktion mit Wasserstoffperoxid Percarbonsäuren oder Percarbonsäureanionen bilden kann.

8. Verfahren nach Anspruch 7, dadurch gekennzeichnet, daß man als Bleichaktivator mindestens eine organische Verbindung zusetzt, die N-Acylgruppen, vorzugsweise N-Acetylgruppen, enthält.

9. Verfahren nach Anspruch 8, dadurch gekennzeichnet, daß man als Bleichaktivator Tetraacetylethylendiamin und/oder Tetraacetylglykoluril zusetzt.

10. Verfahren nach mindestens einem der Ansprüche 5 bis 9, dadurch gekennzeichnet, daß man 0,1 bis 5 Gew.-% Bleichaktivator, bezogen auf Waschaktivsubstanz im Neutralisationsprodukt, zusetzt.

11. Verfahren nach mindestens einem der Ansprüche 1 bis 10, dadurch gekennzeichnet, daß man das Wasserstoffperoxid und gegebenenfalls Bleichaktivator enthaltende Neutralisationsprodukt so lange einer temperaturkontrollierten Nachreaktion unterwirft, bis es eine Klett-Farbzahl ≦, vorzugsweise ≦ 100 (gemessen in einer 5 Gew.-% Waschaktivsubstanz enthaltenden wäßrigen Lösung in einer 4 cm-Küvette mit Blaufilter 420 nm) aufweist.

12. Verfahren nach mindestens einem der Ansprüche 1 bis 11, dadurch gekennzeichnet, daß man dem Neutralisationsprodukt vor der Nachreaktion weitere 0,1 bis 2 Gew.-%, vorzugsweise 0,1 bis 0,5 Gew.-% Wasserstoffperoxid, bezogen auf vorhandene Waschaktivsubstanz, zusetzt.

13. Verfahren nach mindestens einem der Ansprüche 1 bis 12, dadurch gekennzeichnet, daß man bei der Neutralisation das Wasserstoffperoxid gleichzeitig mit dem Sulfierprodukt und der wässrigen Alkalimetallhydroxidlösung unter Aufrechterhaltung eines pH-Wertes im Bereich von 2 bis 8 in eine 0 bis 70 Gew.-% Waschaktivsubstanz enthaltende wässrige Phase einleitet und Waschaktivsubstanzgehalte von 60 bis 70 Gew.-%, vorzugsweise 60 bis 65 Gew.-%, einstellt.

14. Verfahren nach Anspruch 13, dadurch gekennzeichnet, daß die wässrige Phase am Anfang 0 Gew.-% Waschaktivsubstanz enthält.

15. Verfahren nach mindestens einem der Ansprüche 13 und 14, dadurch gekennzeichnet, daß man in der wässrigen Phase bis zum Erreichen eines Gehaltes an Waschaktivsubstanz von 55 bis 65 Gew.-%, vorzugsweise von 60 bis 65 Gew.-%, einen pH-Wert im Bereich von 2 bis 6, vorzugsweise im Bereich von 3 bis 5, aufrechterhält.

16. Verfahren nach mindestens einem der Ansprüche 13 bis 15, dadurch gekennzeichnet, daß man in der wässrigen Phase nach dem Erreichen eines Gehaltes an Waschaktivsubstanz von 55 bis 65 Gew.-%, vorzugsweise von 60 bis 65 Gew.-%, einen pH-Wert im Bereich von 5 bis 8, vorzugsweise im Bereich von 5,5 bis 7,5, aufrechterhält.

17. Verfahren nach Anspruch 13, dadurch gekennzeichnet, daß die wässrige Phase am Anfang mindestens 55 Gew.-% α-Sulfofettsäurealkylester-Alkalimetallsalz enthält.

18. Verfahren nach mindestens einem der Ansprüche 13 und 17, dadurch gekennzeichnet, daß man in der wässrigen Phase einen pH-Wert im Bereich von 5 bis 8, vorzugsweise im Bereich von 5,5 bis 7,5 aufrechterhält.

19. Verfahren nach mindestens einem der Ansprüche 1 bis 18, dadurch gekennzeichnet, daß man die Neutralisation bei Temperaturen unterhalb von 95 °C, vorzugsweise bei Temperaturen im Bereich von 60 bis 80 °C, durchführt.

20. Verfahren nach mindestens einem der Ansprüche 1 bis 19, dadurch gekennzeichnet, daß man den pH-Wert der wässrigen Phase durch Variation der Volumenströme von α-Sulfofettsäurealkylester und wässriger Alkalimetallhydroxidlösung steuert.

21. Verfahren nach mindestens einem der Ansprüche 1 bis 20, dadurch gekennzeichnet, daß man als Ausgangsmaterial Ester von Fettsäuren mit 10 bis 18 Kolenstoffatomen und gesättigten aliphatischen Alkoholen mit 1 bis 4 Kohlenstoffatomen einsetzt.

22. Verfahren nach mindestens einem der Ansprüche 1 bis 21, dadurch gekennzeichnet, daß man als Ausgangsmaterial Fettsäuremethylester einsetzt.

23. Verfahren nach mindestens einem der Ansprüche 1 bis 22, dadurch gekennzeichnet, daß man als Ausgangsmaterial durch Umesterung von natürlichen Fetten und/oder Ölen mit Methanol erhältliche Fettsäuremethylestergemische einsetzt.

24. Verfahren nach mindestens einem der Ansprüche 1 bis 23, dadurch gekennzeichnet, daß man die Umsetzung mit SO₃ in einem Molverhältnis Fettsäureester : SO₃ im Bereich von 1 : 1,2 bis 1 : 1,8 durchführt.

25. Verfahren nach mindestens einem der Ansprüche 1 bis 24, dadurch gekennzeichnet, daß man das aus dem Sulfierreaktor kommende rohe Sulfierprodukt in einer geeigneten Vorrichtung unter mechanischer Bewegung einer Nachreaktion bei 60 bis 100°C bis zum Erreichen eines Sulfiergrades von mindestens 90 %, vorzugsweise von 94 bis 98 % überläßt.

## Claims

1. A process for the production of light-colored pastes of α-sulfofatty acid alkyl ester alkali metal salts by reaction of fatty acid alkyl esters with gaseous SO₃, subsequent after-reaction in liquid phase, neutralization with aqueous alkali metal hydroxide solutions and treatment with hydrogen peroxide, characterized in that the hydrogen peroxide is added to the α-sulfofatty acid alkyl ester during neutralization and the neutralization product obtained is subjected to a temperature-controlled after-reaction.

2. A process as claimed in claim 1, characterized in that 0.5 to 3% by weight and preferably 1.5 to 2.5% by weight hydrogen peroxide, based on washing-active substance in the neutralization product, is added during neutralization.

3. A process as claimed in at least one of claims 1 and 2, characterized in that the hydrogen peroxide is used in the form of a 5 to 35% by weight solution and preferably in the form of a 10 to 20% by weight solution.

4. A process as claimed in at least one of claims 1 to 3, characterized in that the neutralization product is subjected to an after-reaction at 60 to 90°C.

5. A process as claimed in at least one of claims 1 to 4, characterized in that at least one bleach activator is added during neutralization.

6. A process as claimed in at least one of claims 1 to 4, characterized in that at least one bleach activator is added after neutralization and before the temperature-controlled after-reaction.

7. A process as claimed in claim 5 or 6, characterized in that at least one organic compound which is capable of forming percarboxylic acids or percarboxylic acid anions with hydrogen peroxide under the conditions of the neutralization reaction and the temperature-controlled after-reaction is added as the bleach activator.

8. A process as claimed in claim 7, characterized in that at least one organic compound containing N-acyl groups, preferably N-acetyl groups, is added as bleach activator.

9. A process as claimed in claim 8, characterized in that tetraacetyl ethylenediamine and/or tetraacetyl glycoluril is added as bleach activator.

10. A process as claimed in at least one of claims 5 to 9, characterized in that 0.1 to 5% by weight bleach activator, based on the washing-active substance in the neutralization product, is added.

11. A process as claimed in at least one of claims 1 to 10, characterized in that the the neutralization product containing hydrogen peroxide and optionally bleach activator is preferably subjected to a temperature-controlled after-reaction until it has a Klett color value of ≦ 200 and preferably ≦ 100 (as measured on an aqueous solution containing 5% by weight washing-active substance in a 4 cm cuvette with a blue filter at 420 nm).

12. A process as claimed in at least one of claims 1 to 11, characterized in that another 0.1 to 2% by weight and preferably another 0.1 to 0.5% by weight hydrogen peroxide, based on the washing-active substance present, is added to the neutralization product before the after-reaction.

13. A process as claimed in at least one of claims 1 to 12, characterized in that, during neutralization, the hydrogen peroxide is introduced together with the sulfonation product and the aqueous alkali metal hydroxide solution into an aqueous phase containing 0 to 70% by weight washing active substance at a pH value in the range from 2 to 8 and washing active substance contents of 60 to 70% by weight and preferably 60 to 65% by weight are established.

14. A process as claimed in claim 13, characterized in that the aqueous phase initially contains 0% by weight washing-active substance.

15. A process as claimed in at least one of claims 13 and 14, characterized in that a pH value in the range from 2 to 6 and preferably in the range from 3 to 5 is maintained in the aqueous phase until a content of washing-active substance of 55 to 65% by weight and preferably 60 to 65% by weight is reached.

16. A process as claimed in at least one of claim 13 to 15, characterized in that a pH value in the range from 5 to 8 and preferably in the range from 5.5 to 7.5 is preferably maintained in the aqueous phase after a washing-active substance content of 55 to 65% by weight and preferably 60 to 65% by weight has been reached.

17. A process as claimed in claim 13, characterized in that the aqueous phase initially contains at least 55% by weight α-sulfofatty acid alkyl ester alkali metal salt.

18. A process as claimed in at least one of claims 13 and 17, characterized in that a pH value in the range from 5 to 8 and preferably in the range from 5.5 to 7.5 is maintained in the aqueous phase.

19. A process as claimed in at least one of claims 1 to 18, characterized in that the neutralization is carried out at temperatures below 95°C and preferably at temperatures in the range from 60 to 80°C.

20. A process as claimed in at least one of claims 1 to 19, characterized in that the pH value of the aqueous phase is controlled by variation of the feed rates of α-sulfofatty acid alkyl ester and aqueous alkali metal hydroxide solution.

21. A process as claimed in at least one of claims 1 to 20, characterized in that esters of fatty acids containing 10 to 18 carbon atoms and saturated aliphatic alcohols containing 1 to 4 carbon atoms are used as the starting material.

22. A process as claimed in at least one of claims 1 to 21, characterized in that fatty acid methyl ester is used as the starting material.

23. A process as claimed in at least one of claims 1 to 22, characterized in that fatty acid methyl ester mixtures obtainable by transesterification of natural fats and/or oils with methanol are used as the starting material.

24. A process as claimed in at least one of claims 1 to 23, characterized in that the reaction with SO₃ is carried out in a molar ratio of fatty acid ester to SO₃ of 1 : 1.2 to 1 : 1.8.

25. A process as claimed in at least one of claims 1 to 24, characterized in that the crude sulfonation product coming from the sulfonation reactor is subjected to a temperature-controlled after-reaction in a suitable apparatus, in which it is mechanically agitated, at 60 to 100°C until a degree of sulfonation of at least 90% and preferably 94 to 98% is reached.

## Revendications

1. Procédé de production de pâtes de couleur claire en sels de métaux alcalins-alkylesters d'acides gras alpha-sulfoniques par mise en réaction d'alkylesters d'acides gras avec du SO₃ gazeux, suivie de réaction postérieure en phase liquide, neutralisation par des solutions aqueuses d'hydroxyde de métal alcalin et traitement par de l'eau oxygénée, caractérisé en ce que l'on ajoute l'eau oxygénée pendant la neutralisation des alkylesters d'acides gras alpha-sulfoniques et que l'on soumet le produit de neutralisation obtenu à une réaction postérieure thermostatisée.

2. Procédé selon la revendication 1, caractérisé en ce que l'on ajoute lors de la neutralisation 0,5 à 3 % en poids, de préférence 1,5 à 2,5 % en poids, d'eau oxygénée par rapport à la substance détergente-active dans le produit de neutralisation.

3. Procédé selon au moins une des revendications 1 et 2, caractérisé en ce que l'on ajoute l'eau oxygénée comme solution à 5 à 35 % en poids, de préférence à 10 à 20 % en poids.

4. Procédé selon au moins une des revendications 1 à 3, caractérisé en ce que l'on soumet le produit de neutralisation à une réaction postérieure à 60 à 90 °C.

5. Procédé selon au moins une des revendications 1 à 4, caractérisé en ce que l'on ajoute au moins un activateur de blanchiment pendant la neutralisation.

6. Procédé selon au moins une des revendications 1 à 4, caractérisé en ce que l'on ajoute au moins un activateur de blanchiment après la fin de la neutralisation et avant la réaction postérieure thermostatisée.

7. Procédé selon la revendication 5 ou 6, caractérisé en que l'on ajoute comme activateur de blanchiment, au moins un composé organique, qui peut former des acides percarboxyliques ou des anions d'acides percarboxyliques dans les conditions de la neutralisation et de la réaction postérieure thermostatisée.

8. Procédé selon la revendication 7, caractérisé en ce que l'on ajoute comme activateur de blanchiment, au moins un composé organique qui comporte des groupes N-acyle, de préférence des groupes N-acétyle.

9. Procédé selon la revendication 8, caractérisé en ce que l'on ajoute comme activateur de blanchiment de la tétraacétyléthylènediamine et/ou du tétraacétylglycolurile.

10. Procédé selon au moins une des revendications 5 à 9, caractérisé en ce que l'on ajoute 0,1 à 5 % en poids d'activateur de blanchiment, par rapport à la substance détergente-active dans le produit de neutralisation.

11. Procédé selon au moins une des revendications 1 à 10, caractérisé en ce que l'on soumet le produit de neutralisation renfermant l'eau oxygénée et, le cas échéant, l'activateur de blanchiment, à une réaction postérieure thermostatisée jusqu'à ce qu'il présente un indice de coloration de Klett ≦ 200, de préférence ≦ 100 (déterminé dans une solution aqueuse renfermant 5 % en poids de substance détergente-active dans une cuvette de 4 cm avec un filtre bleu de 420 nm).

12. Procédé selon au moins une des revendications 1 à 11, caractérisé en ce que l'on ajoute encore au produit de neutralisation avant la réaction postérieure 0,1 à 2 % en poids, de préférence 0,1 à 0,5 % en poids d'eau oxygénée, par rapport à la substance détergente-active présente.

13. Procédé selon au moins une des revendications 1 à 12, caractérisé en ce que l'on introduit lors de la neutralisation l'eau oxygénée en même temps que le produit de sulfuration et que la solution aqueuse d'hydroxyde de métal alcalin, en maintenant un pH situé dans l'intervalle de 2 à 8, dans une phase aqueuse renfermant 0 à 70 % en poids de substance détergente-active et que l'on ajuste des concentrations en substance détergente-active de 60 à 70 % en poids, de préférence de 60 à 65 % en poids.

14. Procédé selon la revendication 13, caractérisé en ce que la phase aqueuse renferme initialement 0 % en poids de substance détergente-active.

15. Procédé selon au moins une des revendications 13 et 14, caractérisé en ce que l'on maintient dans la phase aqueuse un pH dans l'intervalle de 2 à 6, de préférence dans l'intervalle de 3 à 5, jusqu'à obtention d'une concentration en substance détergente-active de 55 à 65 % en poids, de préférence de 60 à 65 % en poids.

16. Procédé selon au moins une des revendications 13 à 15, caractérisé en ce que l'on maintient dans la phase aqueuse un pH dans l'intervalle de 5 à 8, de préférence dans l'intervalle de 5,5 à 7,5, après obtention d'une concentration en substance détergente-active de 55 à 65 % en poids, de préférence de 60 à 65 % en poids.

17. Procédé selon la revendication 13, caractérisé en ce que la phase aqueuse renferme initialement au moins 55 % en poids de sel de métal alcalin-alkylester d'acide gras alpha-sulfonique.

18. Procédé selon au moins une des revendications 13 et 17, caractérisé en ce que l'on maintient dans la phase aqueuse un pH dans l'intervalle de 5 à 8, de préférence de 5,5 à 7,5.

19. Procédé selon au moins une des revendications 1 à 18, caractérisé en ce que l'on opère la neutralisation à des températures inférieures à 95 °C, de préférence à des températures dans l'intervalle de 60 à 80 °C.

20. Procédé selon au moins des revendications 1 à 19, caractérisé en ce que l'on ajuste le pH de la phase aqueuse en faisant varier les flux volumiques de l'alkylester d'acide gras alpha-sulfonique et de la solution aqueuse d'hydroxyde de métal alcalin.

21. Procédé selon au moins une des revendications 1 à 20, caractérisé en ce que l'on met en oeuvre comme matière de départ des esters d'acides gras comportant 10 à 18 atomes de carbone et d'alcools aliphatiques saturés comprenant 1 à 4 atomes de carbone.

22. Procédé selon au moins une des revendications 1 à 21, caractérisé en ce que l'on met en oeuvre comme matière de départ de l'ester méthylique d'acide gras.

23. Procédé selon au moins une des revendications 1 à 22, caractérisé en ce que l'on met en oeuvre comme matière de départ, des mélanges d'esters méthyliques d'acides gras obtenables par transestérification de graisses et/ou d'huiles naturelles avec du méthanol.

24. Procédé selon au moins une des revendications 1 à 23, caractérisé en ce que l'on opère la réaction avec le SO₃ dans un rapport molaire ester d'acide gras:SO₃, situé dans l'intervalle de 1:1,2 à 1:1,8.

25. Procédé selon au moins une des revendications 1 à 24, caractérisé en ce que l'on soumet le produit brut de sulfuration venant du réacteur de sulfuration dans un dispositif approprié, sous agitation mécanique, à une réaction postérieure à 60 à 100 °C jusqu'à obtention d'un degré de sulfuration d'au moins 90 %, de préférence de 94 à 98 %.
